# EUROPEAN PATENT APPLICATION

(11) **EP 1 690 499 A2**
(43) Date of publication of application: **16.08.2006**
(21) Application number: 06100957.7
(22) Date of filing: 27.01.2006
(51) Int. Cl.: A61B 17/04, A61B 17/16

(54) **Apparatus for attaching sutures**

(30) Priority: 10.02.2005 US 55551
(71) Applicant: Inion Oy, 33520 Tampere (FI)
(72) Inventor: Pierce, Javin, Stowe VT Vermont 05672 (US); Karhi, Olli, 90100, Oulu (FI); Vuorisalo, Vesa, 33530, Tampere (FI); Pirhonen, Eija, 33180, Tampere (FI); Happonen, Harri, 33820, Tampere (FI)
(74) Representative: Kaukonen, Juha Veikko

(57) **Abstract**

A suture anchor (1), an insertion tool (Fig 4) for inserting a suture anchor, and a device for internal working of hole in bony tissue (Fig 5). The anchor comprises an anchor body having a longitudinal cross-section defined by a perimeter. The perimeter comprises at least one tissue-intruding edge (4) formed between the first side and the second side of the perimeter. The intruding edge is arranged to penetrate the tissue during a rotating motion of the anchor in said opening. The anchor further comprises an abutment surface (5), arranged substantially on the opposite side of the anchor body in relation to said intruding edge. The first side of the intruding edge is arranged to connect with the abutment surface by a connecting part of the perimeter having the shape of a substantially curved piece (8) in said longitudinal cross-section.

## Description

### BACKGROUND OF THE INVENTION

The invention relates to an apparatus for attaching sutures to a human tissue. More particularly, the invention relates to a suture anchor made of preferably biodegradable material absorbing into the body, an insertion tool having an insertion end for inserting and attaching the anchor to a tissue, and a device for internal working of a hole in live human tissues.

Suture anchors for attaching a suture to a bony tissue so that another body tissue, e.g. ligament or muscle, may be sutured to the bony tissue are known. Such anchors, as well as devices that are used to attach anchors to a bony tissue, are disclosed, for example, in US Patent Nos. 5 522 844, 5 540 718, 5 683 418, 5 807 403, 6 007 566 and 6 183 479, each of which are hereby incorporated by reference in their entireties for all purposes.

However, known suture anchors suffer from several disadvantages. The structure of the anchor may be vulnerable to breakage due to mechanical stresses affecting the anchor during its fixation to tissue. For example, eyelet failures have been reported to originate from stresses that are caused when sutures attached to the anchor are pulled taut. Sutures can become twisted during the insertion of screw type anchors, hindering the completion of the repair in most conventional anchors, the suture eyelet is a small ring, or a hole that forms a pulley surface to enable suture running. These conventional anchors can suffer from problems including suture running friction due to the small radius of the pulley aspect of the eyelet, and suture damage due to sharp portions of the anchor near the suture running path. Furthermore, the pull-out strength, i.e. the force needed to pull the anchor out of the hole where it has been fixed, may be quite low. These shortcomings, of course, limit the application of the anchor. The insertion and fixation procedure of the anchor may be complicated and include several steps requiring extreme intentness by the operator executing the operation. Furthermore, known tools and devices that are used to attach anchors to a bony tissue may be unpractical from the standpoint of the operator.

### SUMMARY OF THE INVENTION

It is an object of the present invention to provide a suture anchor that is particularly useful for attaching sutures to a human tissue. This objective is achieved by providing a suture anchor attaching one or more sutures in an opening within a tissue, wherein the anchor is attached to said tissue by rotating the anchor relative to said opening, said suture anchor comprising a anchor body having a longitudinal cross-section defined by a perimeter, the perimeter comprising at least one intruding edge formed between the first side and the second side of the perimeter, the intruding edge being arranged to intrude into the tissue during a rotating motion of the anchor in said opening, an abutment surface, arranged substantially on the opposite side of the anchor body in relation to said intruding edge, wherein the first side of the intruding edge is arranged to connect with the abutment surface by a connecting part of the perimeter having the shape of a substantially curved piece in said longitudinal cross-section.

An advantage is that the anchor attaches firmly to the tissue, due to the rotational movement and the co-operation of the intruding edge and the abutment surface. Another advantage is that because the intruding edge and the abutment surface are connected together by the connecting part of the perimeter having a substantially continuous cross-section, the operator may rotate the anchor smoothly and in a controlled way from its insertion position to its attaching position.

According to one embodiment of the present invention, the anchor comprises at least one slot for at least one suture, said slot being arranged transversal in respect of the longitudinal cross-section, and being connected to the perimeter by a passageway. An advantage is that one or more sutures may be threaded at its intermediate portion to the slot, which makes it easier to attach sutures to the anchor, and can enable said suture to be first permanently connected to a ligature, and/or another anchor, greatly increasing the procedural options for the surgeon, and eliminating the need for suture "shuttle" devices.

According to one embodiment of the present invention, the perimeter of the anchor body comprises a protruding part at the perimeter that is arranged on the other side of the perimeter in relation to the connecting part. An advantage is that the protruding part establishes a point of support against the wall of the opening, which point of support induces the anchor to begin its rotation towards a position where it will be attached to the tissue.

It is another object of the present invention to provide an insertion tool for inserting a suture anchor in an opening within a tissue. This objective is achieved by providing an insertion tool comprising an elongated body having a first end and a second end, an insertion end arranged to the first end, a frame arranged outside of the elongated body, the frame comprising a holder sleeve, for receiving and holding the anchor, the body being arranged movably in its longitudinal direction in relation to the frame so that said insertion end is capable of moving through said holder sleeve, said insertion tool further comprising: means for moving said body in said longitudinal direction in relation to said frame, means for attaching suture arranged to the suture anchor arranged in said holder sleeve, said means for attaching sutures being arranged movably in respect of the frame, wherein said means for attaching suture are arranged to move with the suture at least substantially the same distance as the anchor when said anchor is pushed out of the holder sleeve.

An advantage of the insertion tool is that it simplifies the insertion and attaching procedure because both the anchor and the suture can be controlled with it through the whole insertion operation.

It is still another object of the present invention to provide a device for internal working of hole in bony tissue which is particularly useful for working a previously drilled hole in a bony tissue for receiving a suture anchor that is reoriented after insertion to be securely positioned inside the bony tissue. This objective is achieved by providing a device comprising: an elongated body having a first end and a second end, a working end arranged to the first end, a sleeve arranged to surround at least a part of the elongated body, the elongated body being arranged movably in relation to the sleeve in its longitudinal direction, the traveling length of the elongated body being at least distance D, the working end comprising at least one blade or compactor, arranged at the distal end of said working end, the sleeve comprising a tip having an outer circumference and arranged to envelop the working end, the tip comprising an opening, wherein the blade is arranged to have two positions in respect of said tip so that in a first position the blade is arranged to situate substantially inside of the circumference of the tip, and in a second position at least a part of the blade is arranged to situate substantially outside of the circumference of the tip, the device further comprising means for moving the blade from said first position to said second position when the anchor body is moved with said distance D in relation to the frame.

An advantage is that by means of the device it is possible to produce holes, e.g. in bony tissue, which has an internally situated extension part. Said extension part can facilitate the rotation of a suture anchor from its insertion position to an attaching position.

These and other objects, features and advantages of the present invention will become apparent from the following description taken in conjunction with the accompanying drawings.

### BRIEF DESCRIPTION OF THE FIGURES

Figure 1 is a schematic side view of a suture anchor according to the present invention,

Figures 2a is a schematic perspective view of another suture anchor according to the present invention,

Figure 2b is a schematic front view of the suture anchor shown in Figure 2a,

Figure 2c is a schematic side view of the suture anchor shown in Figure 2a,

Figure 2d is a schematic top view of the suture anchor shown in Figure 2a,

Figure 2e is a schematic cross-sectional view of the suture anchor shown in Figure 2a,

Figure 3 is a schematic side view of an insertion end of an insertion tool according to the present invention,

Figures 4a is a schematic side view of an insertion tool according to the present invention,

Figure 4b is a schematic cross-sectional view of a detail of the insertion tool shown in Figure 4a,

Figure 4c is a second schematic side view of an insertion tool shown in Figure 4a,

Figure 4d is a second schematic cross-sectional view of a detail of the insertion tool shown in Figure 4a,

Figure 5a is a schematic side view of a device for internal working of hole in tissue according to the present invention,

Figure 5b is a schematic cross-sectional view of a detail of the device shown in Figure 5a,

Figure 5c is a second schematic cross-sectional view of a detail of the device shown in Figure 5a,

Figures 6a to 6d are schematic representations of a method for attaching a suture to a bony tissue, wherein said method is carried out by a suture anchor, an insertion tool and a device according to the present invention, and

Figure 7 is a schematic perspective view of another suture anchor according to the present invention.

In the figures, the invention is shown simplified for the sake of clarity.

### DETAILED DESCRIPTION OF CERTAIN PREFERRED EMBODIMENTS

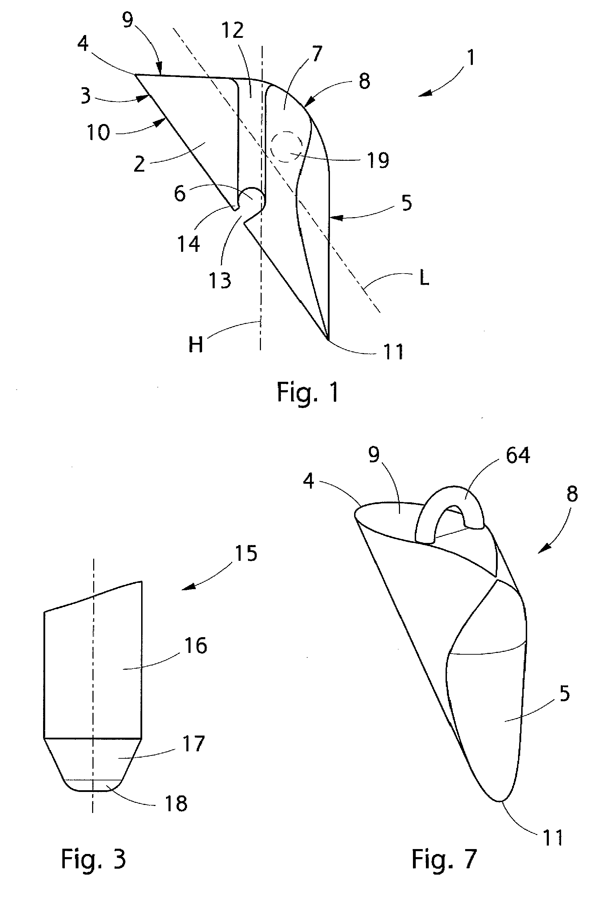

Figure 1 is a schematic side view of a suture anchor according to the present invention.

The suture anchor comprises an anchor body 2, which is preferably made of a biodegradable polymer material that absorbs into the organ system and is prepared by polymerising or copolymerising for instance lactic acid, L-lactide, D-lactide, D,L-lactide, mesolactide, glycolic acid, glycolide or a cyclic ester copolymerised with lactide, or of any other corresponding material known per se to a person skilled in the art, which will not be discussed in this context in greater detail. Other suitable biodegradable polymers, copolymers and polymer mixtures are listed in the following publications, for instance:
- *"Encyclopedic Handbook of Biomaterials and Bioengineering, Part A,"* Donald, L. Wise, Debra J. Trantolo, David E. Altobelli, Michael J. Yaszemski, Joseph D. Gresser, Edith R. Schwartz, 1992, by Marcel Dekker, Inc., pages 977 to 1007,
- *"Biodegradable fracture-fixation devices in maxillofacial surgery,"* R. Suuronen, lnt. J. Oral Maxillofac. Surg., 1993, 22: 50 to 57,
- *"Critical Concepts of Absorbable Internal Fixation,"* William S. Pietrzak, Portland Bone Symposium, Portland, Oregon, August 4 to 7, 1999,
- "*High-impact poly(L*/*D-lactide) for fracture fixation: in vitro degradation and animal pilot study,*" Jan Tams, Cornelis A.P. Joziasse, Ruud R.M. Bos, Fred R. Rozema, Dirk W. Grijpma and Albert J. Pennings, Biomaterilas 1995, Vol. 16, No. 18, pages 1409 to 1415,
- *"A Review of Material Properties of Biodegradable and Bioresorbable Polymers and Devices for GTR and GBR Applications*," Dietmar Hutmacher, Markus B. Hbrzeler, Henning Schliephake, The International Journal of Oral & Maxillofacial Implants, Volume 11, Number 5, 1996, pages 667 to 678, and
- "*Orthopaedic Application for PLA-Pga Biodegradable Polymers,*" Kyriacos A. Athanasiou, Mauli Agrawal, Alan Barber, Stephen S. Burkkhart, The Journal of Arthroscopic and Related Surgery, Vol. 14, No. 7 (October), 1988: 726 to 737.

Further, it is obvious to a person skilled in the art that the material can be a composite that contains two or more materials or monomers, polymer chains, having the essential property of dissolving in the system. A composite can contain bio-glass, bio-ceramics, biologically active components, a pharmaceutical, such as antibiotic or growth factor, or the like.

Further, the material of the anchor body 2 may comprise softeners, such as a pyrrolidone plasticizer. The pyrrolidones useful in the implants or methods of the present invention are any pyrrolidones known in the art of chemistry to have plasticizing properties without having tissue impairing effects or toxic effects. Such pyrrolidones include alkyl- or cycloalkyl-substituted pyrrolidones, such as N-methyl-2-pyrrolidone (NMP), 1-ethyl-2-pyrrolidone (NEP), 2-pyrrolidone (PB), and 1-cyclohexyl-2-pyrrolidone (CP), with NMP and NEP being preferred examples.

The material of the anchor body 2 can be dyed with a suitable coloring agent to improve the visibility of the anchor. The coloring agent can for instance be D&C Green No. 6, chemical name 1,4-bis[(4-methylphenyl)amino]-9,10-anthracenedion (CAS No. 128-80-3) or D&C Violet No. 2, chemical name 1,4-hydroxy[(4-methylphenyl)amino]-9,10-anthracenedion (CAS No. 81-48-1), both coloring agents accepted and named by FDA (Food and Drug Administration). D&C Green No. 6 has obtained FDA approval (21CFR74.3206) for use in dyeing biodegradable sutures used in general surgery or ophthalmic surgery. D&C Violet No. 2 is approved (21CFR74.3602) for use in various sutures and biodegradable meniscus clamps made of a poly(L-lactic acid) material. The amount of the coloring agent in the finished anchor is at most approximately 0.03%, preferably 0.002 to 0.02%.

The material of the anchor body 2 can also contain biologically active agents, such as growth factors, that can promote the healing of the tissue especially in the area of the fixation opening. Biologically active agents are selected from the group consisting of anti-inflammatory agents, antibacterial agents, antiparasitic agents, antifungal agents, antiviral agents, anti-neoplastic agents, analgesic agents, anaesthetics, vaccines, central nervous system agents, growth factors, hormones, antihistamines, osteoinductive agents, cardiovascular agents, anti-ulcer agents, bronchodilators, vasodilators, birth control agents, fertility enhancing agents and polypeptides. Preferably the bioactive agents are bone morphogenic proteins (BMP), such as OP-1, BMP-2, BMP-4 and BMP-7. The biologically active agents can be incorporated, for instance, via coatings or mixing them with biodegradable materials of the anchor body 2.

It should be noted that the suture anchor is hereafter on referred to as 'anchor'. The anchor body 2 may be formed by any conventional molding process, such as injection molding or compression molding, or by any other commonly known forming method, such as machining, used to form products from polymer materials. The anchor body 2 defines a perimeter 3, which has a nearly triangular shape in the embodiment shown in Figure 1. At one corner of the anchor body 2 there is an intruding edge 4. The intruding edge 4 is formed between a first side 9 and a second side 10 of the anchor body 2. The angle between said first and second side 9 and 10 is now approximately 52°. Said angle can be for instance within the range of 30° to 70°, preferably 40° to 65°, more preferably 40° to 55°.

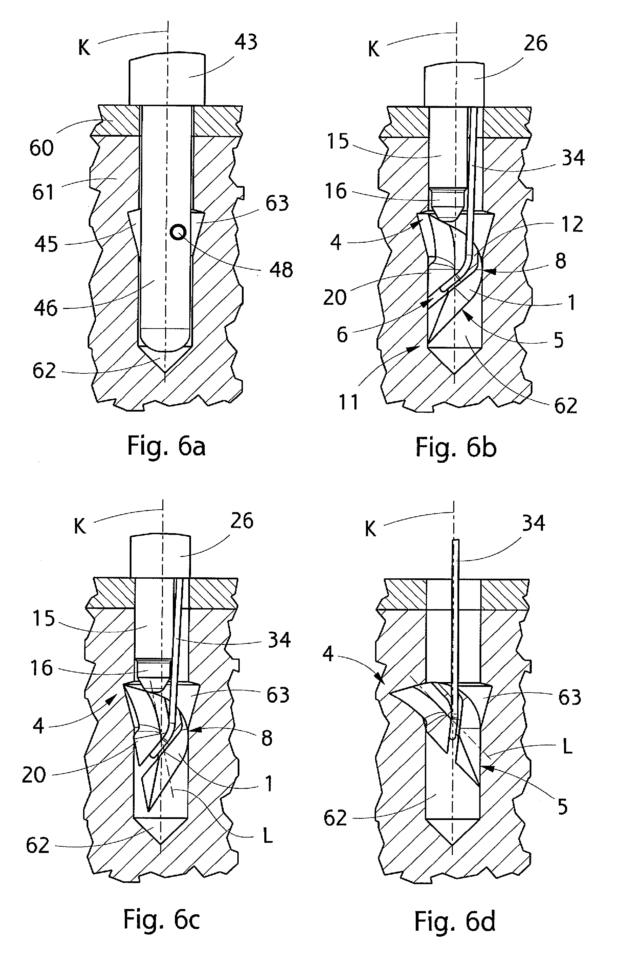

The function of the intruding edge 4 is to intrude or penetrate into the tissue where the anchor 1 is inserted in order to attach the anchor 1 to said tissue. For example, the anchor 1 may be inserted into a predrilled hole in bony tissue. During the insertion the anchor 1 has pushed its way into said hole in a position where its longitudinal axis L is at least substantially parallel with the longitudinal axis of said predrilled hole, and where the tip 11 of the anchor 1 is foremost. In said predrilled hole, the anchor 1 is induced to change angle or rotate relative to the longitudinal axis of said hole in retrograde motion caused by pulling sutures arranged to the anchor 1. It is to be noted here that the insertion procedure is described more detailed in connection with Figures 6a to 6d. During the rotation, the intruding edge 4 is penetrating to bony tissue forming the wall of said predrilled hole. The anchor 1 rotates in the hole until it is induced to stop rotation and the anchor 1 becomes firmly affixed in the bony tissue. Then the anchor 1 has a position where the longitudinal axis of the hole is about parallel with axis H shown in Figure 1. In the embodiment shown in Figure 1, the rotational angle is approximately 35°, but it can be for instance within the range of 20° to 55°, preferably 30° to 45°. Due to the relative wide angle of which the anchor 1 is rotated in the hole or another type of opening, the pull-out strength of the anchor 1 is exceptionally high.

Furthermore, the anchor body 2 comprises an abutment surface 5, which is arranged on an opposite side of the anchor body 2 in relation to said intruding edge 4. The function of the abutment surface 5 is to communicate with the wall of the opening, e.g. a predrilled hole in bony tissue, and interact with the intruding edge 4 to affix the anchor 1 firmly in the opening.

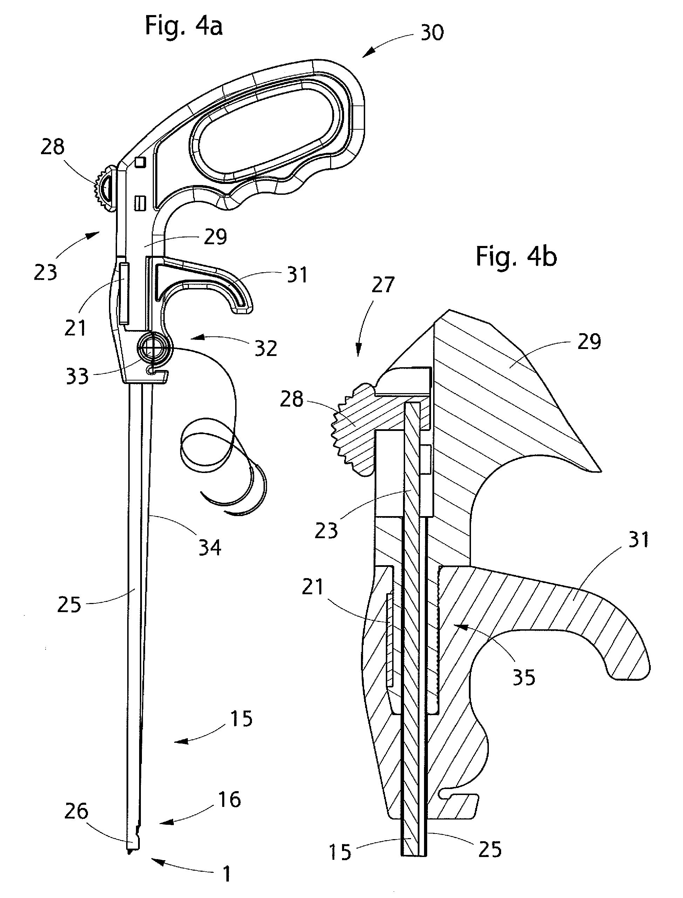

The first side 9 of the perimeter is connected with the abutment surface 5 by a curvilinear connecting part 8 so that the anchor body 2 turns smoothly and gradually from the first side 9 to the abutment surface 5. The curvilinear shape of the connecting part causes the anchor 1 to translate and rotate smoothly, evenly and in a controlled way against the wall of the opening and an insertion tool during its rotational movement in the opening. It is to be noted that the insertion tool is discussed more detailed in connection with Figures 3 and 4a to 4d.

The abutment surface 5 forms a tip 11 with the second side 10. The tip 11 can be used as penetration tip in those embodiments of application of the anchor 1, for instance, where the anchor 1 is to be inserted into soft tissue and/or where there is no pre-made hole or opening in said tissue. It is to be noted that the tip 11 is not an obligatory feature of the anchor 1 but it can be replaced with, for example, a rounded or truncated end.

A slot 6 is defined in the anchor body 2. The slot 6 is arranged transversal in respect of the longitudinal cross-section of the anchor body 2 and it extends between a first flank surface 7 and a second flank surface of the anchor body. It is to be noted that only the first flank surface 7 is in view in Figure 1; the second flank surface is behind the anchor body 2 and therefore out of sight. The slot 6 is connected to the perimeter 3 by a passageway 13. One or more sutures can be arranged in the slot 6. Because the slot 6 communicates with the perimeter 3, there is no need to thread any suture end through any small passageways in the anchor 1. Instead, an intermediate portion of suture thread may be threaded in the slot 6 which is a far simpler procedure to carry out, and enables novel surgical methods where the suture can be permanently affixed to the body or devices, at one or both ends, before the anchor 1 is attached to the suture. If needed, the suture can be tied to the anchor 1 by a knot.

The anchor 1 may also comprise several slots 6, for example two or three slots 6. This kind of structure is especially useful if there is a need to arrange more than one suture in the anchor 1, e.g. when the tissue to be sewn to the anchor 1 by sutures is fragile or delicate. The anchor body 2 of the anchor is robust and, therefore, the anchor 1 withstands the stresses caused to it during the insertion and fixation operation.

There is a lip 14 arranged at the passageway 13. The lip 14 reduces the width of the passageway 13 smaller than that of the slot 6. Thanks to the lip 14, the suture arranged into the slot 6 remains there, or at least the probability that the suture will escape from the slot 6 is reduced. The structure of the lip 14 can be flexible so that when the suture is threaded to the slot 6 the lip 14 yields under the pressure caused by the tautened suture.

The anchor body 2 also comprises two grooves 12 formed in the flank surfaces and extending from the slot 6 to the perimeter 3, more precisely to the first side 9 of the perimeter in the embodiment shown in Figure 1. The grooves 12 are provided to permit the protected passage of the suture out of the hole or opening in tissue. Furthermore, the grooves 12 are arranged to guide the sutures in the way that the tension induced by user on the sutures causes the anchor to rotate from an insertion position, i.e. the position where the anchor 1 has been inserted in the opening, to an attaching position, i.e. the position where the anchor 1 has been attached firmly in the opening. The shape of the grooves and their end points at the perimeter can differ from those shown in Figure 1. It is to be noted here that grooves 12 are not obligatory but the anchor 1 can also be realized without any grooves 12 extending between the slot 6 and the perimeter 3.

The anchor body 2 can comprise one or more drug reservoirs 19. The anchor 1 in Figure 1 comprises one drug reservoir 19 that is depicted with dashed lines. The drug reservoir can be, for example, a blind hole that is filled with a bioactive agent. The bioactive agent is selected from the group consisting of anti-inflammatory agents, antibacterial agents, antiparasitic agents, antifungal agents, antiviral agents, anti-neoplastic agents, analgesic agents, anaesthetics, vaccines, central nervous system agents, growth factors, hormones, antihistamines, osteoinductive agents, cardiovascular agents, anti-ulcer agents, bronchodilators, vasodilators, birth control agents, fertility enhancing agents and polypeptides. Preferably the bioactive agents are bone morphogenic proteins (BMP), such as OP-1, BMP-2, BMP-4 and BMP-7.

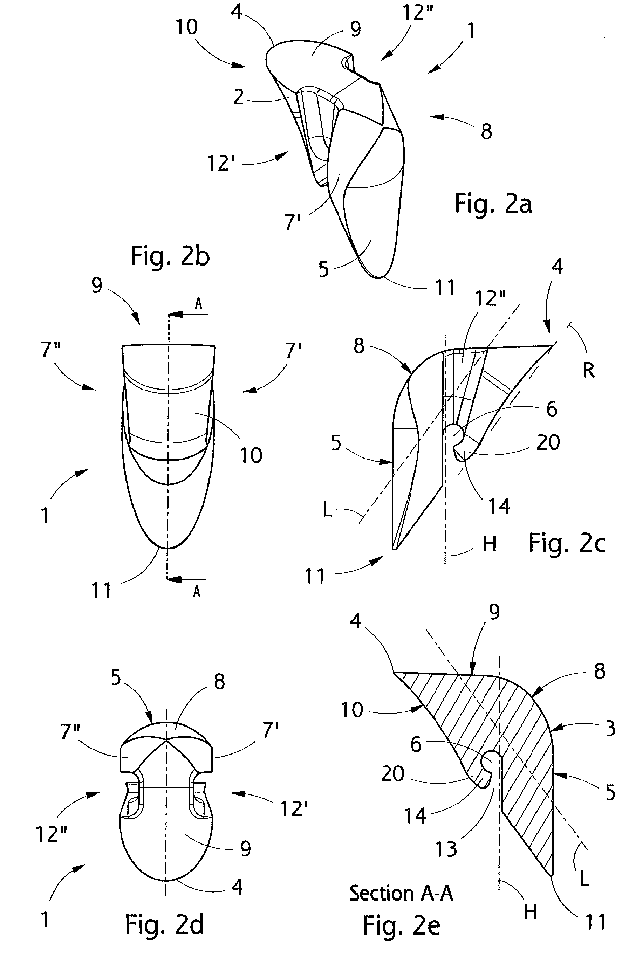

Figures 2a is a schematic perspective view of another suture anchor according to the present invention, Figure 2b is a schematic front view of the suture anchor shown in Figure 2a, Figure 2c is a schematic side view of the suture anchor shown in Figure 2a, Figure 2d is a schematic top view of the suture anchor shown in Figure 2a, and Figure 2e is a schematic cross-sectional view of the suture anchor shown in Figure 2a.

The anchor body 2 defines a perimeter 3 which has a triangular shape where one corner is substituted by a curved shaped connecting part 8. At one corner of the perimeter 3 there is an intruding edge 4. An intruding edge 4 is formed between a first side 9 and a second side 10 of the perimeter 3. The intruding edge 4 has a curved, shovel-like shape that is clearly shown in Figure 2d. It is to be noted that the intruding edge 4 may have another shape, for example, it can be straight, or comprise two or more intruding tips or teeth, etc.

The tip 11 also has a curved shape as shown in Figure 2b, but of course, it can have some other shape. It can, for example, include one or more sharp pikes.

As shown in Figure 2d, the cross-section of the abutment surface 5 has a rounded shape. The radius of the rounded shape is preferably set to fit the radius of the bore hole into which the anchor 1 is indented to be inserted. It is clear that the cross-section of the abutment surface 5 can also be shaped otherwise. For example, it can have a planar shape.

A slot 6 for one or more sutures is arranged in the anchor body 2. The slot 6 extends between a first flank surface 7' and second a flank surface 7" of the anchor body 2. The slot 6 is connected to the perimeter 3 by a passageway 13. At one side of the passageway 13 there is a lip 14. The lip 14 is arranged to restrict the cross-section of the passageway 13 as discussed already in connection with Figure 1. The lip 14 makes a projection 20 that projects outwards in the perimeter 3. A line or plane surface R that is drawn via the intruding edge 4 and the outmost point of the projection 20 shows that there is a concave section of the perimeter 3 between the intruding edge 4 and the projection 20. Due to the concave section, the rotation of the anchor 1 from its insertion position to its locking position can begin around the projection 20. The projection 20 establishes a bearing surface against the wall of the opening, around which bearing surface the anchor 1 is arranged to begin its rotation towards a stabilized locking position. This is discussed more detailed in connection with Figures 6b to 6d. The concave section can also assist the penetration of the intruding edge 4 into the tissue.

The projection can also be placed otherwise, i.e. it is not obligatory to place it in the lip 14. Nevertheless, the projection 20 should be arranged with respect to the connecting part 8 on the opposite side of the anchor body 2 so as to facilitate the rotation of the anchor 1 from its insertion position to its locking position.

The connecting part 8 has a curved, convex shape. The curve can comprise just one turning radius through the whole connecting part 8, or alternatively, it can include two or even more sections having different turning radii. It is also possible that the connecting part 8 is built from a series of plane surfaces so that adjacent plane surfaces are connected to each other in small angles. The plane surfaces establish a convex surface that connects the first surface 9 to the abutment surface 5 essentially continuously and smoothly. The overall radius of the curved shape of the connecting part 8, i.e. the radius of a circular arch corresponding to the convex shape of the connecting part 8, and the maximum length of the anchor 1 has preferably a ratio of 0.2:1 - 0.4:1, more preferably 0.23:1 - 0.35:1. The maximum length of the anchor 1 is the maximum length of the anchor 1 measured in the direction of longitudinal axis L. Furthermore, the overall radius of the curved shape of the connecting part 8, and the maximum width of the anchor 1 has preferably a ratio 0.6:1 - 1.2:1, more preferably 0.7:1 - 0.9:1. The maximum width of the anchor 1 is the maximum distance between the connecting part 8 or the abutment surface 5 and the second side 10 in a plane perpendicular to the longitudinal axis L. An anchor 1 dimensioned as above rotates especially smoothly from its insertion position to its locking position.

There are numerous indications where anchors according to the invention can be put to use. For example, the present invention can be used in shoulder for rotator cuff repair, bankart repair, slap lesion repair, biceps tenodesis, acromio-clavicular separation repair, deltoid repair, capsular shift or capsulolabral reconstruction; in foot or ankle: laterial stabilization, medial stabilization, achilles tendon repair, hallux valgus reconstruction, midfoot reconstruction, metatarsal ligament repair; in knee: medial collateral ligament repair, lateral collateral ligament repair, patellar tendon repair, posterior oblique ligament repair, iliotibial band tenodesis; in hand or wrist: scapholunate ligament reconstruction, ulnar collateral ligament reconstruction, radial collateral ligament reconstruction; in elbow: biceps tendon reattachment, tennis elbow repair, ulnar or radial collateral ligament reconstruction; in pelvis: bladder neck suspension for female urinary incontinence due to urethral hypermobility or intrinsic sphincter deficiency. The present invention can also be used for meniscus repair where anchors are used to coapt soft tissue to soft tissue, or for skin closures and plastic surgery, or in any soft tissue to soft tissue, soft tissue to bony tissue, or bony tissue to bony tissue fixation. Because the present invention can readily be affixed to both soft and hard tissues, it can be used in place of suture ligatures where such ligatures are difficult, or time consuming to form, for example in Nissen Fundoplication (GRDS), forming anastomosis, and multiple applications that will be evident to those skilled in the art.

The fixation strength of the anchors shown in Figures 2a to 2e were tested. The anchors were machined from extruded P(L/DL)LA (Purac, Holland) rod which had a diameter of 3.0 mm. After manufacturing the anchors were packed into an aluminium pouch and samples were further gamma sterilized with minimum dose of 25 kGy.

High strength PE Dyneema fibre (HercuLine®, High performance Fishing line: 100% Dyneema Fiber, Sufix USA, Inc, USA) was used as suture in order to achieve the ultimate fixation strength of the anchor. The Sawbones solid rigid polyurethane foam blocks (Pacific Research Laboratories, Inc. WA, USA) with pcf value 30 were used as the model-bone in fixation strength testing.

Ten anchors were placed in foam and immersed into water of temperature of 37 °C for a minimum of 1 hour (the immersion time for all samples was within a range of 1 - 2 hours). Parallel holes were drilled into each foam block at a distance of at least 10 mm from each other and the edges of the foam block with a product-specific 3.5 mm drill bit in straight angle to the surface of the foam block.

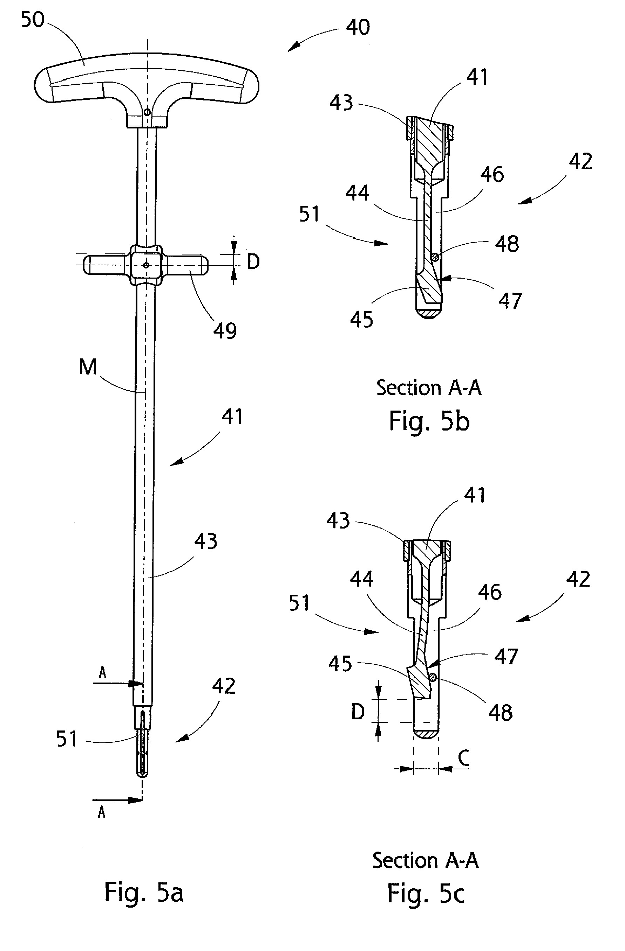

The anchors were inserted into the drill holes by using a device for internal working of hole in tissue shown in Figures 5a to 5c and an insertion tool shown in Figures 4a to 4d. The device was introduced into the drill hole before the insertion of the anchor to create a cavity for the intruding edge of the anchor. After the insertion of the anchor with the tool, force was applied to the sutures manually in order to rotate the anchor to its optimal position so that the anchor was "locked" into the drill-hole wall.

Mechanical testing was performed using a Zwick Z020/TH2A universal materials testing machine (Zwick Gmbh & Co, Ulm, Germany) with a 500-N load cell (class 1). Mechanical testing was performed at 37 °C in water bath. One suture anchor at a time was connected to the testing machine by fixing the foam block under a specially designed clamp, and the ends of the suture were tied together with several knots (flat and square ties with additional throws, i.e., 2x1x1x1x1x1x1x1x1x1x1) to form a tight suture loop over a metal rod connected to the load cell of the testing machine. The initial distance between the metal rod and the surface of the foam block, i.e. drill hole entrance, was approximately 5 cm. The fixation strength of each anchor was tested by pulling the sutures parallel with the long axis of the drill hole at the constant speed of 60 mm/min. The specimen's response to the loading was obtained automatically in the form of a load-displacement curve. The maximum failure load (N) and failure mode were recorded. The test results are shown in Table 1.

**Table 1**

| Number of specimen | Fixation strength [N] | Failure mode |
|---|---|---|
| 1 | 243.68 | Suture cut through anchor |
| 2 | 223.19 | Suture cut through anchor |
| 3 | 237.55 | Suture cut through anchor |
| 4 | 237.79 | Suture cut through anchor |
| 5 | 211.72 | Suture cut through anchor |
| 6 | 234.99 | Anchor edge bent and anchor pulled out from the hole |
| 7 | 237.33 | Suture cut through anchor |
| 8 | 232.85 | Suture cut through anchor |
| 9 | 176.63 | Anchor edge bent and anchor pulled out from the hole |
| 10 | 238.19 | Anchor edge bent and anchor pulled out from the hole |

The average fixation strength was 227.39 N with a standard deviation of 20.06 N. The anchor shown in Figures 2a - 2e has superior fixation strength values compared to, for example, Panalok anchor (Mitek Products, Westwood, MA). In commercial sense, Panalok anchor is one of the most successful suture anchors available at present. In an article (Dominik C. Mayer et al., "Mechanical Testing of Absorbable Suture Anchors", in Arthroscopy Vol 19, No 2 (2003): pp. 188-193.) the fixation strength test for Panalok anchor gave an average load to failure value of 133 N.

Figure 3 is a schematic side view of an insertion end of an insertion tool according to the present invention. The insertion tool includes an elongated body 15 having a first end and a second end. It is to be noted that only the first end 16 of the body 15 is shown in Figure 3. The elongated body 15 can be solid or hollow and it can be made of suitable metal or plastic material. The cross-section of the body 15 as well as of the first end 16 is circular, but their shape may vary. At the end of the first end 16 there is an insertion end 17, and at the very end of the insertion end there is an end surface 18. The overall shape of the insertion end 17 is a truncated cone where the edges of the cone surface and the end surface 18 are rounded. During the retrograde rotational movement of the anchor in an opening in tissue the end surface 18 has a contact with the first side of the perimeter of the anchor. The end surface 18 can also have a contact with the connecting part of the anchor during said rotational movement.

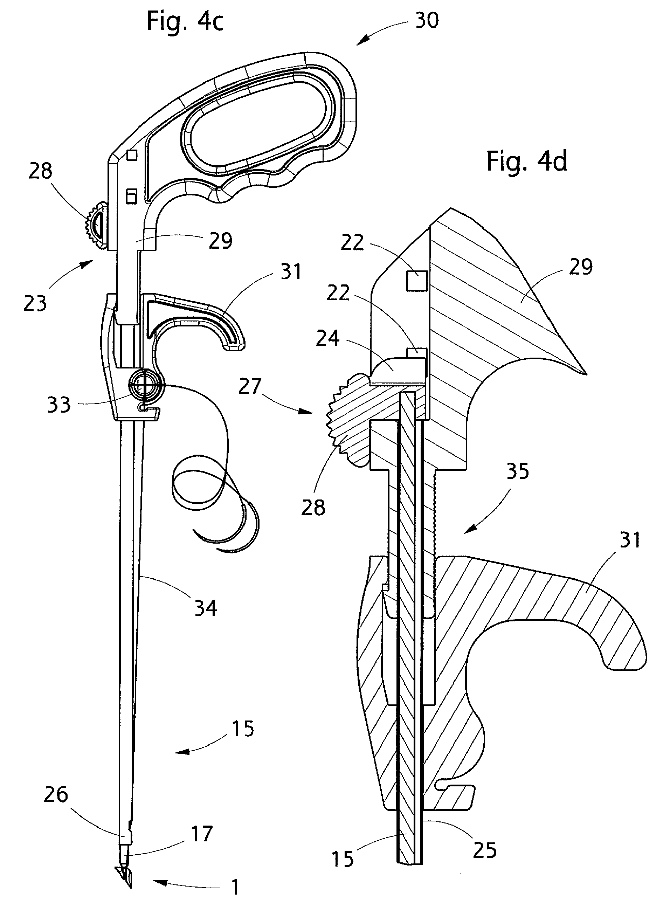

Figure 4a is a schematic side view of an insertion tool according to the present invention, Figure 4b is a schematic cross-sectional view of a detail of the insertion tool shown in Figure 4a, Figure 4c is a second schematic side view of an insertion tool shown in Figure 4a, and Figure 4d is a second schematic cross-sectional view of a detail of the insertion tool shown in Figure 4a.

The insertion tool comprises an elongated body 15 having a first end 16 and a second end 23. The cross-section of the elongated body can be, for example, circular. An insertion end 17 is arranged to the first end 16. There is shown one example of the structure of the insertion end 17 in Figure 3. Naturally, the shape of the insertion end 17 can differ from the shapes shown in Figure 3.

A frame 25 is arranged outside of the elongated body 15. The frame 25 has a tubular structure, inside of which the elongated body 15 is arranged. The frame 25 comprises a holder sleeve 26 at its distal end. The holder sleeve 26 can receive and hold at least one suture anchor 1.

The elongated body 15 is arranged inside of the frame 25 so that it can be moved or glided in the longitudinal direction of the frame 25 in relation to the frame 25. In the situation shown in Figures 4a and 4b, the elongated body 15 is in a retracted position. This means that the insertion end 17 is retracted away from the holder sleeve 26, thus leaving space in the holder sleeve 26 to receive the anchor 1.

The movement between the elongated body 15 and the frame 25 is accomplished by means 27 for moving the elongated body. In the embodiment shown in Figures 4a to 4d, said means 27 comprises a slide button 28 that is attached at the second end 23 of the elongated body 15. The user of the insertion tool can slide the slide button 28 to and fro in relation to the body 29 of the insertion tool. Because the frame 25 is attached to the body 29, the movement of the slide button 28 makes the elongated body 15 to slide to and fro, respectively, inside of the frame 25. As the insertion end 17 is pushed through the holder sleeve 26, the anchor 1 being held in said sleeve 26 is forced out from within the sleeve 26.

The body 29 of the insertion tool comprises a handle 30 that facilitates the proper use of the tool.

The insertion tool comprises further means 32 for attaching one or more sutures to the tool. In the embodiment shown in Figures 4a to 4d, said means 32 consists of a cleat 33 that comprises slots arranged crosswise in the longitudinal direction of the cleat 33. Sutures to be attached to the tool are jammed in said slots. The cleat 33 is attached to a grip 31. The grip 31 is, on the other hand, arranged movably in relation to the body 29 of the tool so that the grip 31 and the cleat 33 can be moved in the longitudinal direction of the frame 25. An array of ridges 35 is arranged between the body 29 and the grip 31. The array of ridges 35 causes convenient friction that opposes the movement between the body 29 and the grip 31. It is to be noted that the means for 32 for attaching sutures to the tool can be designed otherwise. Also the array of ridges 35 can be replaced with other structures that cause friction when the grip 31 is moving with respect to the body 29.

In Figures 4a and 4b, the grip 31 as well as the elongated body 15 are in their extreme retracted position with respect to the body 29. In Figure 4b there is shown a safety catch 21. The safety catch 21 prevents unintentional movements of the grip 31 with relative to the body 29. The safety catch 21 will be removed from its place before the grip 31 is moved in the relation to the body 29.

The anchor 1 is arranged inside of the holder sleeve 26 and the suture 34 attached to the anchor is jammed in the receiving space of the cleat 33 so that the suture is tensioned between the anchor and the cleat 33. Then, the user of the tool guides the holder sleeve 26 at the opening inside of which the anchor 1 is to be inserted. It is to be noted that the opening is not shown in Figures 4a to 4d. As the holder sleeve 26 is aligned with the opening in a proper way, the user begins to glide the slide button 28 towards the holder sleeve 26. By means of the insertion end 17 the user pushes the anchor 1 out from within the holder sleeve 26. As the anchor 1 penetrates into the opening, the tensioned suture 34 pulls the grip 31 towards the holder sleeve 26. When the anchor 1 has reached the right position, i.e. depth, in the opening, the position of the slide button 28 and the elongated body 15 on the one hand and the grip 31 on the other hand can be similar to those shown in Figures 4c and 4d. Now the user begins to pull the grip 31 in the opposite direction, i.e. towards the handle 30. Because of the tensioned suture 34, the movement of the grip 31 causes a retrograde motion to the anchor 1 inside of the opening. Due to the design of the anchor 1 it begins to slide against the insertion end 17 of the elongated body and rotate from its insertion position to its locking position. The intruding edge 4 of the anchor 1 is intruding into tissue during the rotational movement.

The elongated body 15 is restrained from a retrograde motion. The body 29 of the tool comprises notches or cavities 22 whereas projections 24 are arranged in the button 28. The projection 24 can snap in the notch 22 and thereby lock the button 28 and the elongated body 15 in a definite position in relation to the body 29 and the frame 25. The locking between the notch 22 and the projection 24 can be released simply by bending the projection 24 out of the notch 22.

As the anchor 1 has reached its locking position, the suture 34 is released from the cleat 33.

Figures 5a to 5c are schematic representations of a device for internal working of hole in bony tissue. The device 40 comprises an elongated body 41 having a first end and a second end and having longitudinal axis M. A working end 42 is arranged to the first end of the body 41, and a first handle 49 is arranged in the second end of the body 41.

The device 40 comprises a tubular sleeve 43 which is arranged to surround the elongated body 41. A second handle 50 is arranged in one end of the sleeve 43. The body 41 is arranged movably in relation to the sleeve 43 in the direction of the longitudinal axis M, i.e. the body 41 can slide to and fro inside of the sleeve 43. Due to this, the working end 42 can be moved in relation to the sleeve 43. The traveling length of the body 41 is at least distance D with respect to the sleeve 43.

When examining the structure of the working end 42, it is detected that it comprises a flexible shank 44 made of a flexible material. Said material can be, for example, stainless steel, Nitinol or some other metallic alloy or plastic. The shank 44 is an integrated part of the body 41 and made of the same material with it. Alternatively, the shank 44 is a separate component that has been attached to the body 44 and made of the same or a different material compared to the body 44.

Additionally, the working end 42 comprises a blade 45 arranged at the distal end of said shank 44. The blade 45 has a cutting edge that is capable of cutting at least cancellous bone. The blade 45 is integrated into the shank 44, but it is also possible that the blade 45 is a component separate from the shank 44 and attached to the shank 44 detachably or undetachably. Instead of the blade 45 there can be a blunt compacting element arranged in the working end 42.

The sleeve 43 comprises a tip 46 having an outer circumference, the diameter of which is marked by reference mark C in Figure 5c. The tip 46 is arranged around the working end 42. The tip 46 comprises an opening 51, through which the blade 45 can move out of the circumference of the tip 46, and again back inside of the circumference C.

The blade 45 is arranged to have two positions in respect of said tip 46, the first of which is shown in Figure 5b. The blade 45 is substantially inside of the circumference C of the tip 46 in the first position. The second position of the blade 45 is shown in Figure 5c. In the second position, at least a part of the blade 45 is situated substantially outside of the circumference C of the tip 46.

The blade 45 is moved from the first position to the second position by pulling the body 41 and the working end 42 attached thereto distance length D upwards, in the directions shown in Figures 5a to 5c. The operator who is operating the device 40 makes the blade 45 move from the first position to the second position by pulling the first handle 49 by distance D towards the second handle 50. As the body 41 moves upwards in respect of the sleeve 43 and the tip 46 attached thereto, the diagonal back surface 47 of the blade 45 contacts with a pin 48 attached to the tip 46. The pin 48 forces the blade 45 to move outwards from the first position to the second position, i.e. from the position shown in Figure 5b to the position shown in Figure 5c. The use of the device 40 is described more detailed in the part of this description relating to Figure 6a.

The profile of the blade 45 may vary depending of the application of the device. Instead of a cutting blade 45, it is also possible to apply a compressing blade that does not cut or remove the tissue being worked, but compresses it tightly around the hole or opening. It is also possible to apply a blade that partially compresses, partially cuts the tissue being worked.

Figures 6a to 6d are schematic representations of a method for attaching a suture to a bony tissue, wherein said method is carried out by a suture anchor, an insertion tool and a device according to the present invention.

Prior to the method step shown in Figure 6a, a borehole 62 is formed through hard cortical bone 60 to soft cancellous bone 61. This can be done with a drill, for instance, or some other instrument. Such instruments are known per se, so they are not discussed in more detail herein. The imaginary longitudinal center axis of the borehole 62 is marked by reference symbol K.

After this, in the method step shown in Figure 6a, a device for internal working of hole in bony tissue is arranged to the borehole 62. One example of such a device is shown in Figures 5a to 5c. More promptly, the tip 46 of the device is inserted within the borehole 62. When the insertion takes place, the blade 45 is within the tip 46, i.e. in the first position.

The insertion of the device is continued until the sleeve 43 of the device is in contact with the surface of the cortical bone 60. After this, the blade 45 is moved outside of the circumference of the tip 46, i.e. the blade 45 is moved in the second position shown in Figure 6a. Next, the device is rotated around the longitudinal center axis of the tip 46, the blade being in its second position. The sleeve 43 of the device bears against the cortical bone 60 ensuring that the device is well supported during the rotation.

Upon rotation of the tip 46 the blade 45 cuts an extension part 63 of the borehole to the cancellous bone 61. In Figure 6a, the tip 46 has been turned a full circle, i.e. at least 360°, about the longitudinal center axis of the tip 46. Therefore, the extension part 63 is a rotationally symmetrical space around the borehole 62. It is clear that the extension part 63 can also be made so that the tip 46 is not turned a full circle but less about the longitudinal center axis of the tip 46. In this way it is possible to made an extension part 63 that is non-symmetrical in relation to the borehole 62.

After the extension part 63 has been made, the blade is moved back to its first position and the tip 46 is removed within the borehole 62.

It is to be noted here that the method step shown in Figure 6a is not obligatory but the anchor 1 can be inserted and attached to a normal borehole without any extension part.

Next, an anchor 1 is inserted within the borehole 62 with the help of an insertion tool. One example of such an insertion tool is shown in Figures 4a to 4d. The anchor 1 is first arranged inside of the holder sleeve 26 of the tool as discussed earlier. When the tool is aligned with the borehole 62 in a proper way, the user pushes the anchor 1 out from within the holder sleeve 26 and inserts the anchor 1 into the borehole, as shown in Figure 6b. The anchor 1 is inserted so deep in the borehole that it has at least partly bypassed the extension part 63. During insertion a suture 34 arranged to the slot of the anchor 1 is kept tight with means for attaching one or more sutures to the insertion tool, such as disclosed in Figures 4a to 4d.

When the anchor 1 has reached the right depth in the borehole 62, the user of the insertion tool begins to tension the suture 34. This can be done, for example, by pulling the suture 34 or, alternatively by keeping the suture tight and pushing the anchor 1 with the elongated body 15 of the insertion tool. In this way the anchor 1 is induced to move retrograde against the first end 16 of the elongated body 15. The retrograde movement induces the anchor 1 to change its angle relative to the borehole 62, see Figure 6c.

The design of the anchor 1 facilitates the rotation of the anchor 1 in the borehole 62. It is to be noted that the rotation takes place counterclockwise in Figures 6b to 6d. Firstly, the anchor 1 shown in Figures 6b to 6c comprises a projection 20 that extends from the tip 11 over the passageway of the suture. The projection 20 forms a bearing surface or fulcrum against the wall of the borehole 62. The bearing surface facilitates the anchor 1 to begin its rotation in the borehole 62. Secondly, grooves 12 are arranged in the anchor 1 to guide the suture 34 from the slot 6 and out of the borehole 62. The grooves 12 are designed so as to cause the tensioned suture 12 to turn the anchor 1 around the bearing surface created by the projection 20. Thirdly, the round shaped connecting part 8 of the anchor 1 slides at first smoothly against the wall of the borehole 62 and then against the first end 16 of the tool. Fourthly, the smoothly shaped upper surface of the anchor 1, i.e. the first side 9 of the perimeter and connecting part 8 can co-operate with the first end 16 of the tool so that there is a fulcrum between the anchor 1 and the first end 16. It is possible that all the features of the anchor 1 facilitating the rotation of the anchor 1 in the borehole do not play a significant role in some specific operations using the anchor 1. Nevertheless, their presence makes the anchor 1 versatile and thereby usable in multiplicity of indications. The retrograde and rotational movement of the anchor induces the intruding edge 4 to penetrate the wall of the borehole 62 in the area of the extension part 63. The extension part 63 can establish on the one hand space for the anchor 1 to rotate at a rather wide angle before the intruding edge 4 interacts with the wall of the borehole; thereby, the intruding edge 4 will penetrate in the tissue surrounding the hole 62 at a relatively wide angle. On the other hand, the extension part 63 can establish corners or edges at which the intruding edge 4 can "grip" easily and begin to penetrate in the tissue.

As the anchor 1 rotates, the connecting part 8 presses against the wall of the borehole 62. The connecting part 8 can be dimensioned so that an increase in the rotation angle correlates with an increase in the pressure between the connecting part 8 and the wall. This increasing pressure can help the intruding edge 4 to penetrate in the tissue on the other side of the borehole.

The anchor 1 continues its rotational movement until the abutment surface 5 of the anchor presses against the wall of the borehole 62. If the user of the insertion tool now pulls the suture 34, the anchor 1 becomes firmly affixed in the tissue. The abutment surface 5 is thus arranged to rest against the wall of the borehole 62 and stabilize the position of the anchor 1 in cooperation with the intruding edge 4 in said borehole.

The longitudinal direction of the anchor 1 has been rotated about 45° during the insertion. The angle depends on the design of the anchor 1 and its size in relation to the dimensions of the borehole 62. The value of the angle can be for instance within the range of 20° to 55°, preferably 30° to 45°.

It is not obligatory to insert the anchor 1 into a borehole. The sharp tip 11 is able to penetrate into soft or weak tissues, such as soft tissue or soft cancellous bone, even if there is no hole at all. The anchor 1 can also be inserted into a man-made hole made by pressing with a spike or the like.

It is to be noted here that two or more anchors 1 can be arranged in the same opening in tissue. Such method is disclosed in US Patent No. 5 405 359, which is hereby incorporated by reference in its entirety for all purposes. Sutures of the anchors 1 in the same opening can be arranged so that they are not attached to more than one anchor 1. The dimensions of the anchors 1 can be matched with the dimensions of the opening to be sufficient to limit or allow the free passage or running of a suture attached to another anchor 1 arranged in the same opening.

Figure 7 is a schematic perspective view of another suture anchor according to the present invention. The structure of the anchor 1 is much the same as that of the anchors discussed in the preceding figures. The anchor 1 comprises an intruding edge 4, a first side 9 of the body of the anchor 1, an abutment surface 5, a connecting part 8 arranged between the first side 9 and the abutment surface 5, and a tip 11. The main difference compared to said anchors is that there is no slot at all for one or more sutures in the anchor. Instead, the means for attaching one or more sutures in the anchor is a loop 64 arranged on the boundary between the first side 9 and the connecting part 8.

The loop 64 can be made of the same material as the body of the anchor 1, or of some other material. Preferably, the loop 64 is made of a piece of suture. Said piece of suture can be attached, for instance, to molten material of the anchor body at the same time as the anchor body is molded, or the piece of suture can be glued in small boreholes or grooves made in the anchor body. The loop 64 can be twisted prior to molding it in the anchor to increase the surface contact and geometry of said suture within the molded anchor body.

The drawings and the related description are only intended to illustrate the idea of the invention. The invention may vary in detail within the scope of the claims.

## Claims

1. A suture anchor for attaching a suture in an opening within a tissue, said anchor comprising:
an anchor body having a longitudinal cross-section defined by a perimeter,
the perimeter comprising at least one tissue-intruding edge formed between the first side and the second side of the perimeter, the intruding edge being arranged to penetrate the tissue during a rotating motion of the anchor in said opening,
an abutment surface, arranged substantially on the opposite side of the anchor body in relation to said intruding edge, wherein
the first side of the intruding edge is arranged to connect with the abutment surface by a connecting part of the perimeter having the shape of a substantially curved piece in said longitudinal cross-section.

2. The anchor as claimed in claim 1, comprising at least one slot for at least one suture, said slot being arranged transversal in respect of the longitudinal cross-section, and being connected to the perimeter by a passageway.

3. The anchor as claimed in claim 2, comprising a lip at the passageway, the lip being arranged to reduce the area of the cross-section of the passageway.

4. The anchor as claimed in claim 1, wherein the perimeter comprises a projection on the second side of the perimeter, the projection establishing a bearing surface against the wall of the opening.

5. The anchor as claimed in claim 3, comprising a lip at the passageway, the lip being arranged to reduce the area of the cross-section of the passageway, and a projection arranged on the lip, the projection establishing a bearing surface against the wall of the opening.

6. The anchor as claimed in claim 1, wherein the anchor body is made of a polymeric material.

7. The anchor as claimed in claim 6, wherein the polymeric material is at least partly bioresorbable.

8. The anchor as claimed in claim 6, wherein the polymeric material is dyed.

9. The anchor as claimed in claim 1, wherein the anchor body is made of material comprising active agents.

10. The anchor as claimed in claim 1, wherein the anchor body comprises a groove extending between the slot and the perimeter.

11. The anchor as claimed in claim 1, wherein the angle between the first side and the second side of the perimeter is in a range of 30° - 70°.

12. The anchor as claimed in claim 1, wherein the anchor body comprises a drug reservoir for active agents.

13. The anchor as claimed in claim 1, wherein the anchor comprises at least one suture arranged into the slot.

14. The anchor as claimed in claim 1, comprising at least one loop for at least one suture, said loop being arranged on the perimeter.

15. The anchor as claimed in claim 14, wherein the loop is made of a piece of suture.

16. An insertion tool for inserting a suture anchor in an opening within a tissue and contributing to the anchoring of said suture in said tissue, the insertion tool comprising:
an elongated body having a first end and a second end,
an insertion end arranged to the first end,
a frame arranged outside of the elongated body,
the frame comprising a holder sleeve, for receiving and holding the anchor,
the body being arranged movably in its longitudinal direction in relation to the frame so that said insertion end is capable of moving through said holder sleeve,
said insertion tool further comprising:
means for moving said body in said longitudinal direction in relation to said frame,
means for releasably attaching suture arranged to the suture anchor arranged in said holder sleeve, said means for releasably attaching sutures being arranged movably in respect of the frame, wherein
said means for releasably attaching suture are arranged to move with the suture at least substantially the same distance as the anchor when said anchor is pushed out of the holder sleeve.

17. The insertion tool as claimed in claim 16, wherein the means for attaching suture is a cleat arranged in the tool.

18. A device for internal working of hole in bony tissue, comprising:
an elongated body having a first end and a second end,
a working end arranged to the first end,
a sleeve arranged to surround at least a part of the elongated body,
the elongated body being arranged movably in relation to the sleeve in its longitudinal direction, the traveling length of the elongated body being at least distance D,
the working end comprising at least one blade or compacting element arranged at the distal end of said working end,
the sleeve comprising a tip having an outer circumference and arranged to envelop the working end, the tip comprising an opening,
wherein the blade or compacting element is arranged to have two positions in respect of said tip so that in a first position the blade or compacting element is arranged to situate substantially inside of the circumference of the tip, and in a second position at least a part of the blade or compacting element is arranged to situate substantially outside of the circumference of the tip, the device further comprising:
means for moving the blade or compacting element from said first position to said second position when the body is moved said distance D in relation to the frame.

19. The device for internal working of hole in bony tissue as claimed in claim 18, wherein the working end comprising a shank made to form a spring, the blade or compacting element being arranged at the distal end of said shank, and means for bending the shank in order to move the blade or compacting element from said first position to said second position when the body is moved said distance D in relation to the frame.

20. The device for internal working of hole in bony tissue as claimed in claim 19, wherein the means for bending the shank comprises a diagonal surface attached to the shank and a counterpart arranged to the tip so that when said body is moved in respect of said sleeve, said diagonal surface is arranged to slide in contact with said counterpart, thus moving the blade or compacting element from said first position to said second position.
